(19) Europäisches Patentamt
European Patent Office
Office européen des brevets

(11) Numéro de publication: **0 037 777**
**B1**

(12) **FASCICULE DE BREVET EUROPEEN**

(45) Date de publication du fascicule du brevet: **28.09.83**

(51) Int. Cl.³: **C 07 C 93/02, A 61 K 31/13**

(21) Numéro de dépôt: **81400528.6**

(22) Date de dépôt: **02.04.81**

# (54) Procédé de préparation de cycloalcoyl propanol amines utiles comme médicaments.

(30) Priorité: **04.04.80 FR 8007718**

(43) Date de publication de la demande:
**14.10.81 Bulletin 81/41**

(45) Mention de la délivrance du brevet:
**28.09.83 Bulletin 83/39**

(84) Etats contractants désignés:
**AT BE CH DE FR GB IT LI LU NL SE**

(56) Documents cités:
**BE - A - 837 320**

**CHEMICAL ABSTRACTS, vol. 69, no. 19, 4 November 1968, page 7170 réf. 76809r Columbus, Ohio, US**
**G. FERRARI et al.: "Beta-Adrenergic blocking drugs. III. 1-Aryloxy- and 1-arylamino-3-amino-2-propanols"**

(73) Titulaire: **PIERRE FABRE S.A.**
**125, rue de la Faisanderie**
**F-75116 Paris (FR)**

(72) Inventeur: **Mouzin, Gilbert, Dr. Chem.**
**21, rue Sainte-Foy**
**F-81100 Castres (FR)**
Inventeur: **Cousse, Henri**
**La Foun de Los Nobios Chemin de Lastinos**
**F-81100 Castres (FR)**
Inventeur: **Rieu, Jean-Pierre**
**10, rue Jean Mermoz**
**F-81100 Castres (FR)**

(74) Mandataire: **Corre, Jacques Denis Paul et al,**
**Cabinet Regimbeau 26, Avenue Kléber**
**F-75116 Paris (FR)**

**EP 0 037 777 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Procédé de préparation de cycloalcoyl propanol amines utiles comme medicaments

La présente invention, réalisée au Centre der Recherches Pierre FABRE, concerne de nouveaux dérivés de cycloalcoyl propanolamines possédant des propriétés β bloquantes, leur procédé de préparation et leur application en tant que médicaments utiles dans le traitement des maladies d'origine cardiovasculaire.

Tous les β bloquants actuellement commercialisés appartiennent à deux types de famille, les (PEA) phényl éthanol amines et les (AOPA) aryl oxy propanol amines:

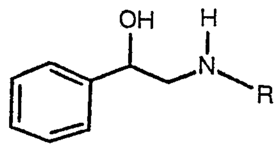

(PEA) et

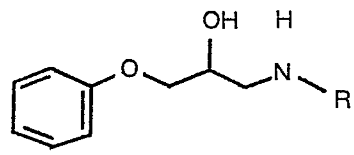

(AOPA)

GADRET et coll. Eur. J. Med. Chem. *4,* 367 (1978) ont montré l'importance fordamentale du noyau aromatique dans ce type de structure.

D'une façon surprenante, la demanderesse a découvert que certaines cycloalcoyl propanolamines dont la structure présente une différence fondamentale avec les PEA et AOPA provenant de l'absence du noyau aromatique, exercent quand même une activité β bloquante.

Certains de ces composés sont déjà connus, en particulier par l'article de G. Ferrari et al., Boll. Chim. Farm. 1968, 107(4), 234—48 et par le brevet belge n° 837 320; toutefois ces documents décrivent des procédés économiquement peu rentables pour la préparation de tels composés, soit parce que la synthèse nécessite plusieurs étapes, soit parce que les températures mises en oeuvre sont assez élevées.

La présente invention a pour objet un procédé de préparation de composés de type cycloalcoyl propanol amines qui présentent de nombreux avantages sur le plan industriel.

L'invention propose un procédé de préparation de composés de formule I:

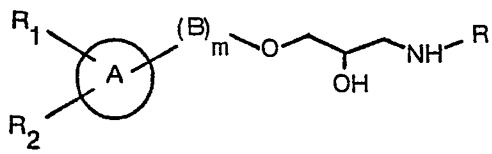

(I)

dans laquelle,

A représente un radical cyclique carboné, non aromatique, comportant au moins un cycle,

$R_1$ représente un radical alcoyle ou acyle,

$R_2$ représente l'hydrogène ou un radical alcoyle,

B représente un radical méthylène,

m est 0, 1 ou 2, et

lorsque m est différent de 0, $R_1$ peut être l'hydrogène,

R représente un radical alcoyle, alcényle, alcynyle ou un radical aralcoyle, et

lorsque R est un radical insaturé, $R_1$ peut être l'hydrogène; caractérisé en ce que l'on fait réagir l'épichlorhydrine sur un alcool de formule IV:

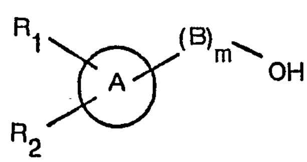

(IV)

dans laquelle $R_1$, $R_2$, A, B et m ont la même signification que dans la formule (I), en présence d'une base en solution aqueuse et d'un catalyseur du type ammonium quaternaire, pour obtenir l'époxyde intermédiaire de formule II:

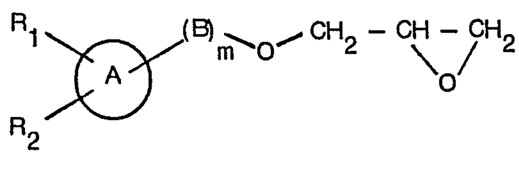

(II)

que l'on fait réagir dans un solvant sur une amine primaire de formule III:

$$R—NH_2 \qquad (III)$$

dans laquelle R à la même signification que pour la formule I, pour obtenir les dérivés cycloalcoylepropanolamines de formule (I).

Dans la formule précédente, le radical A a de préférence de 3 à 10 atomes de carbone et représente de préférence un radical monocyclique comportant de 3 à 6 atomes de carbone saturé, c'est-à-dire cyclopropyl, cyclobutyl, cyclopentyl et cyclohexyl, ou insaturé comportant une ou plusieurs insaturations, en particulier éthylénique, par exemple cyclobutényl, cyclopentényl, cyclohexényl, cyclohexadiényl; mais peut être également un radical bicyclique ou polycyclique qui peut être ponté, c'est-à-dire que les cycles ont au moins 2 atomes de carbone en commun, ou spirannique, c'est-à-dire que les cycles n'ont qu'un atome de carbone en commun; ces cycles pouvant, comme précédemment, présenter une ou plusieurs insaturations.

Par "radical alcoyle" on entend plus particulièrement les radicaux alcoyles inférieurs linéaires ou ramifiés ayant de 1 à 8 atomes de carbone, en particulier de 1 à 4 atomes de carbone, tels que méthyl, éthyl, n-propyl, isopropyl, n-butyl, isobutyl et t-butyl.

Par "radical alcényl" on entend plus particulièrement les radicaux alcényles inférieurs linéaires ou ramifiés ayant de 2 à 8 atomes de carbone et de préférence de 2 à 4 atomes de carbone, tels que éthényl et allyl.

Par "radical alcynyl" on entend plus particulièrement les radicaux alcynyles inférieurs linéaires ou ramifiés ayant de 2 à 8 atomes de carbone et de préférence de 2 à 4 atomes de carbone, tels que propargyl.

Par "radical acyl" $R_1$ on entend le radical correspondant à un acide carboxylique, de préférence un acide carboxylique aliphatique saturé ou non ayant, par exemple, de 2 à 8 atomes de carbone, par exemple acétyl, propionyl, pivaloyl.

Par "radical aralcoyle" on entend désigner des radicaux comportant de préférence de 1 à 3 atomes de carbone dans la partie aliphatique, la partie aryle étant de préférence le radical phényl, par exemple le radical benzyl.

Les radicaux $R_1$ et $R_2$ peuvent être fixés sur le même atome de carbone du cycle ou sur deux atomes de carbone différents.

Des sels des composés deformule I peuventetre préparés avec des par cetteindication acides thérapeutiquement acceptables, on entend désigner des acides minéraux tels que l'acide chlorhydrique, phosphorique et sulfurique ou des acides organiques notamment monocarboxyliques ou dicarboxyliques tels que les acides succiniques, tatriques, oxaliques, maléiques et fumariques.

Dans le cas des polyacides, les sels peuvent être les sels correspondant à la neutralisation de une ou plusieurs des fonctions acides.

Les composés préférés préparés selon la présente invention répondent aux formules I' et I'':

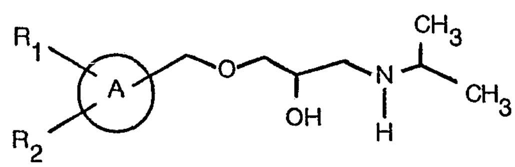

(I')

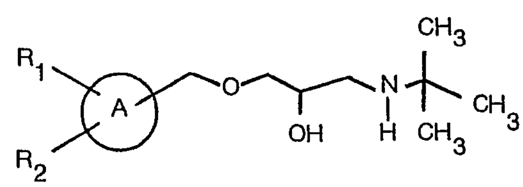

(I'')

Dans le procédé de la présente invention, la réaction de l'époxyde de formule II:

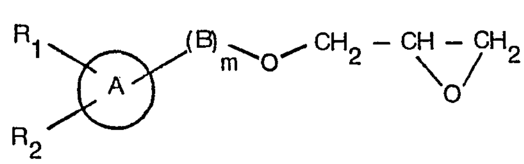

(II)

sur l'maine primaire de formule III:

$$R—NH_2 \qquad (III)$$

est effectuée dans un solvant, en particulier en alcool tel que le méthanol.

Comme cela est indiqué précédemment, l'époxyde de formule II est préparé par action de l'épichlorhydrine sur un alcool de formule IV:

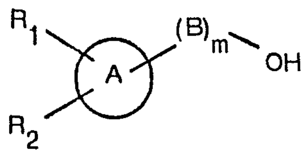

(IV)

en présence d'une base telle que la soude et d'un catalyseur du type ammonium quaternaire par exemple de formule:

$$^{\oplus}N(R_2)_4\ X^-$$

dans laquelle $R_2$ est un radical alcoyle inférieur et $X^-$ est l'anion d'un acide, par exemple $HSO_3^-$ ou $Hal^-$ (Hal:halogène) tel que l'hydrogéno-sulfate de tétrabutylammonium.

Les alcools et les amines mis en oeuvre dans ce procédé sont des composés connus de la technique antérieure.

Les composés préparés par le procédé de la présente invention peuvent être utilisés à titre de médicaments, éventuellement en association avec d'autres principes actifs.

Exemple 1

Préparation du tertiobutylamino-3 (méthyl-2 cyclohexyloxy)-1 propanol-2, maléate

a) Préparation du (méthyl-2 cyclohexyloxy)-1 époxy-2,3 propane

Dans un tricol de 500 ml, on ajoute goutte à goutte en 5 minutes 17,66 g (0,15 mole) de méthyl-2 cyclohexanol à un mélange de 5 g d'hydrogénosulfate de tétrabutylammonium (0,014 mole) dans 80 ml de lessive de soude à 50% et 80 ml d'épichlorhydrine.

L'addition est réalisée de telle façon que la température du milieu réactionnel ne dépasse pas 30°C. Laisser 6 heures à température ambiante, puis ajouter 400 g de glace pilée et extraire à l'éther éthylique.

La phase organique est lavée avec une solution bicarbonatée, puis à l'eau saturée de chlorure de sodium. Après séchage sur sulfate de sodium, filtration et évaporation du solvant, l'huile résiduelle est rectifiée sous vide.

On récupère avec un rendement de 85% l'éther d'époxyde. $Eb_{0,2}$: 58 à 60°C.

b) Préparation du tertiobutylamino-3 (méthyl-2 cyclohexyloxy)-1 propanol-2, maléate

20,6 g (0,28 mole) de tertiobutylamine sont ajoutés à une solution de 12 g (70 mmoles) d'éther d'époxyde précédent dans 120 ml de méthanol. Le mélange réactionnel est maintenu à une température inférieure à 30°C pendant l'addition par refroidissement dans un bain de glace.

Laisser ensuite une nuit sous vive agitation à température ambiante. Evaporer le solvant jusqu'à siccité, extraire à l'éther, laver la phase organique avec une solution bicarbonatée, puis à l'eau saturée de chlorure de sodium.

Sécher sur sulfate de sodium filtrer et traiter par 6 g (0,55 mole) d'acide maléique dans 220 ml d'éther éthylique. Glacer. On récupère après filtration et séchage 70% de produit de formule:

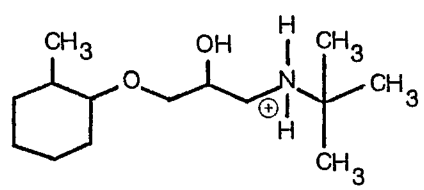

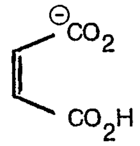

Formule brute: $C_{18}H_{33}NO_6$
Masse moléculaire: 359,4
Cristaux blancs
Point de fusion: 104°C

Chromatographie sur plaque:
- support: gel de silice 60 F 254 Merck
- solvent: chloroforme — méthanol — ammoniaque 80/18/2
- révélation: Iode
- Rf: 0,55

Solubilités: soluble dans l'eau à 10%, dans le diméthyl sulfoxyde à 15%.

Exemple 2

Préparation du tertiobutylamino-3 (isopropyl-2 méthyl-5 cyclohexyloxy)-1 propanol-2, maléate

D'une manière similaire à celle décrite dans l'exemple 1, mais en utilisant le menthol, on obtient le produit de formule:

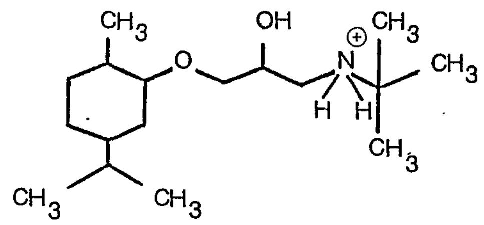

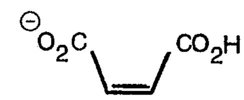

Formule brute: $C_{21}$ $H_{29}$ $NO_6$
Masse moléculaire: 401,5
Cristaux blancs
Point de fusion: 158°C
Chromatographie sur plaque:
- support: gel de silice 60 F 254 Merck
- solvant: chloroforme — méthanol — ammoniaque 80/18/2
- révélation:Iode et réactif de Dragendorff
- Rf: 0,60

Solubilités: soluble dans l'eau à 1%. Soluble dans le diméthyl sulfoxyde à 20%.

Exemple 3

Préparation du (diméthyl-1,1 propyne-2 yl-1) amino-3, cyclohexyloxy-1 propanol-2 oxalate

D'une façon similaire à celle décrite dans l'exemple 1, mais en utilisant le cyclohexanol, la diméthylpropargylamine et l'acide oxalique comme agent salifiant, on obtient le produit de formule:

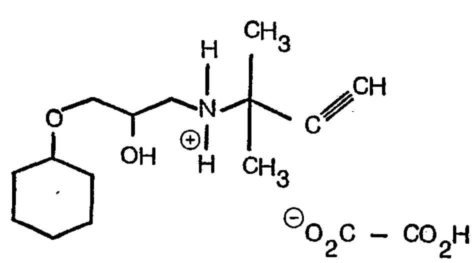

Formule brute: $C_{16}$ $H_{27}$ $NO_6$
Masse moléculaire: 329,4
Cristaux blancs
Point de fusion: 121°C
Spectre de RMN (base libre) ($CDCl_3$): $\delta$ ppm: 3,7 (m, 1H); 3,1 à 3,6 (m, 3H); 2,8 (m, 2H); 2,3 (s, 1H); 1 à 2,1 (m, 16H).
Chromatographie sur plaque:
- support: gel de silice 60 F 254 Merck
- solvent: chloroforme — méthanol — ammoniaque 80/18/2
- révélation: iode
- Rf: 0,8

Solubilités: soluble dans l'eau à 2%.

Exemple 4

Préparation de l'isopropylamino-3, cyclohexyl méthoxy-1 propanol-2 maléate

D'une façon similaire à celle décrite dans l'exemple 1, mais en utilisant le cyclohexylméthanol et l'isopropylamine, on obtient le produit de formule:

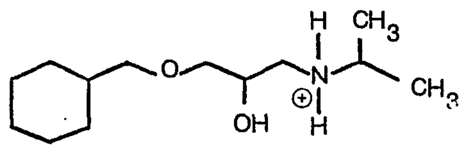

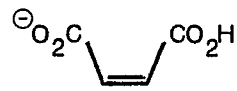

Formule brute: $C_{17}$ $H_{31}$ $NO_6$
Masse moléculaire: 345,4
Cristaux blancs
Point de fusion: 108°C
Spectre de RMN (DMSO $d_6$) $\delta$ ppm 0,5 à 2,2 (m, 18H); 2,6 à 3,7 (m, 7H); 3,9 (m, 1H); 6,1 (s, 2H); 6,3 à 10,5 (4H échangeable)
Chromatographie sur plaque:
— support: gel de silice 60 F 254 Merck
— solvant: chloroforme — méthanol — ammoniaque 80/18/2
— révélation: Iode
— Rf: 0,57
Solubilités: soluble dans l'eau à 10% et à 18% dans le DMSO.

Exemple 5

Préparation du tertiobutylamino-3 cyclohexyl méthoxy-1, propanol-2, maléate

D'une façon similaire à celle décrite dans l'exemple 1, mais en utilisant le cyclohexyméthanol, on obtient le produit de formule:

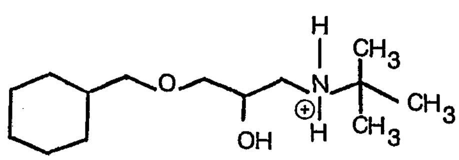

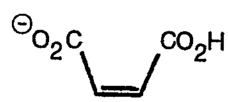

Formule brute: $C_{18}H_{33}NO_6$
Masse moléculaire: 359,4
Cristaux blancs
Point de fusion: 166°C
Spectre de RMN (DMSO $d_6$) $\delta$ ppm: 1,1 à 2 (m, 20H); 2,3 à 3,1 (m, 2H); 3,1 à 3,6 (m, 4H); 3,9 (m, 1H); 6,05 (s, 2H); 5 à 10 (m, 4H échangeables).
Chromatographie sur plaque:
— support: gel de silice 60 F 254 Merck
— solvant: chloroforme — méthanol — ammoniaque 80/18/2
— révélation: Iode
— Rf: 0,50
Solubilités: soluble dans l'eau à 2,5%, dans le diméthyl sulfoxyde à 10%

Exemple 6

Préparation du tertiobutylamino-3 [(diméthyl-6,6, bicyclo (3,1,1) hepten-2 yl)-2 éthoxy]-1 propanol-2, maléate.

D'une façon similaire à celle décrite dans l'exemple 1, mais en utilisant le nopol, on obtient le produit de formule:

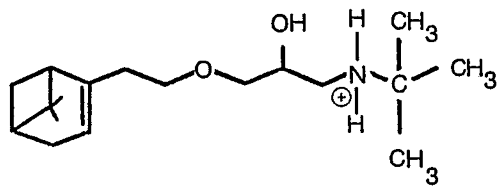

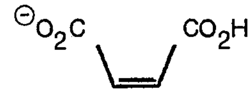

Formule brute: $C_{22} H_{37} NO_6$
Masse moléculaire: 411,5
Cristaux: blancs
Point de fusion: 118°C
Chromatographie sur plaque:
— support: gel de silice 60 F 254 Merck
— solvent: chloroforme — méthanol — ammoniaque 80/18/2
— révélation: Iode
— Rf: 0,61
Solubilités: Soluble dans l'eau à 2%, dans le diméthyl sulfoxyde à 10%.

Exemple 7

Préparation du tertiobutylamino-3 [(pivaloyl-2 cyclopropyl-1) méthoxy]-1 propanol-2, maléate

D'une façon similaire à celle décrite dans l'exemple 1, mais en utilisant le pivaloyl-2 cyclopropyl méthanol, on obtient le produit de formule:

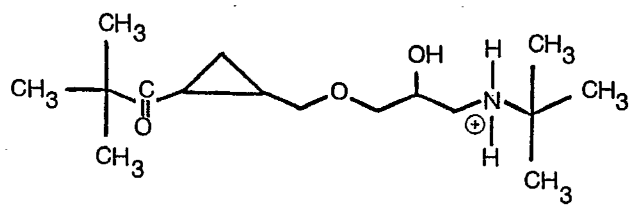

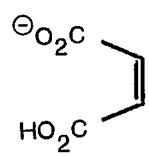

Exemple 8

Préparation du tertiobutylamino-3 (cyclohexen 2 yl-1) méthoxy-1 propanol-2, maléate

D'une façon similaire à celle décrite dans l'exemple 1, mais en utilisant le cyclohexène-3 méthanol, on obtient le produit de formule:

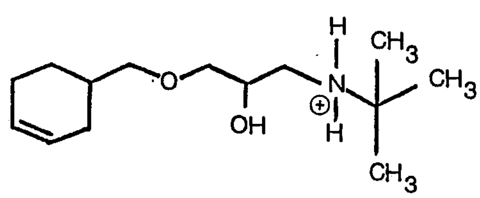

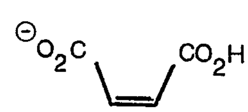

Formule brute: $C_{18} H_{31} NO_6$
Masse moléculaire: 357,4
Cristaux: blancs
Point de fusion : 142°C

Chromatographie sur plaque:

— support: gel de silice 60 F 254 Merck
— solvant: chloroforme — méthanol — ammoniaque 80/18/2
— révélation: Iode et réactif de Dragendorff
— Rf: 0,61

Solubilités: soluble dans l'eau à 10%, dans le diméthylsulfoxyde à 15%.

Exemple 9

Préparation du benzylamino-3 (cyclohexen-3 yl-1) méthoxy-1 propanol-2, maléate

D'une façon similaire à celle décrite dans l'exemple 1, mais en utilisant le cyclohexen-3 méthanol, la benzylamine, on obtient le produit de formule:

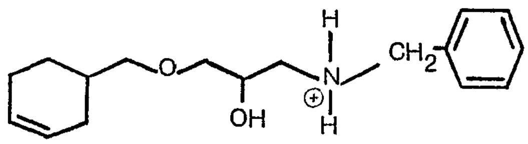

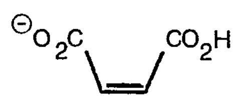

EXPERIMENTATIONS

a) *Toxicologie*

L'étude de la toxicité a été effectuée chez la souris conventionnelle pesant environ 20 g.

Les substances ont été administrées par voie orale ou intra-veineuse. La $DL_{50}$ est calculée selon la méthode de KARBER, Arch. Exptl., Pathol. pharmacol., 1931, *162*, 480.

Par voie intra-veineuse, la $DL_{50}$ est comprise entre 25 et 150 mg/kg.

Par voie orale la $DL_{50}$ est comprise entre 700 et 1 200 mg/kg.

b) *Propriétés pharmacologiques*

Les expérimentations pharmacologiques auxquelles ont été soumis les composés chimiques objet de l'invention ont permis de mettre en évidence d'intéressantes propriétés $\beta$ bloquantes.

Méthode: Tachycardie isoprénalique chez le chien (DUNLOP O. et SHANK P., Brit. J. Pharmacol., 1968, *32*, 201 à 218).

Les produits les plus intéressants réduisent la tachycardie isoprénalique à partir de la dose de 0,3 à, 0,5 mg/kg par voie intra-veineuse.

c) *Applications thérapeutiques*

Compte tenu de leurs propriétés $\beta$ bloquantes et de leur faible toxicité, ces composés peuvent être utilisés en thérapeutique dans le traitement des troubles cardiaques et plus particulièrement dans le traitement de l'hypertension et de l'arythmie cardiaque.

Ces composés peuvent être utilisés sous la forme de compositions pharmaceutiques dans lesquelles on mélange le composé actif avec des diluants ou véhicules non toxiques pharmaceutiquement acceptables facilitant la biodisponibilité des principes actifs.

Ces compositions peuvent être administrées par voie parentérale, intra-veineuse, orale ou rectale.

Les compositions peuvent être par exemple sous la forme de comprimés, de capsules, ge gélules, de suppositoires, de solutions aqueuses ou huileuses, de suspensions aqueuses ou huileuses, d'émulsions, de poudre dispersable ou de solutions aqueuses ou huileuses injectables.

Ces compositions peuvent en particulier se présenter sous des formes galéniques modifiant leur biodisponibilité, soit en favorisant une action rapide, soit au contraire en procurant une action retard.

Les posologies utilisables dépendent du type de composés utilisés, de la voie d'administration et du type d'affection à traiter et peuvent être déterminées par des tests connus.

**Revendications**

1. Procédé de préparation de cycloalcoylepropanolamines répondant à la formule générale I:

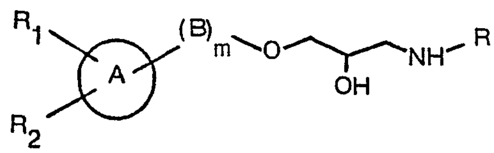

(I)

dans laquelle,

A représente un radical cyclique carboné, non aromatique, comportant au moins un cycle,
$R_1$ représente un radical alcoyle ou acyle,
$R_2$ représente l'hydrogène ou un radical alcoyle,
B représente un radical méthylène,
m est 0, 1 ou 2, et
lorsque m est différent de 0, $R_1$ peut être l'hydrogène,
R représente un radical alcoyle, alcényle, alcynyle ou un radical aralcoyle, et
lorsque R est un radical insaturé, $R_1$ peut être l'hydrogène;

caractérisé en ce que l'on fait réagir l'épichlorhydrine sur un alcool de formule IV:

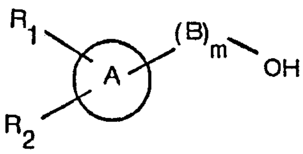

(IV)

dans laquelle $R_1$, $R_2$, A, B et m ont la même signification que dans la formule (I), en présence d'une base en solution aqueuse et d'un catalyseur du type ammonium quaternaire, pour obtenir l'époxyde intermédiaire de formule II:

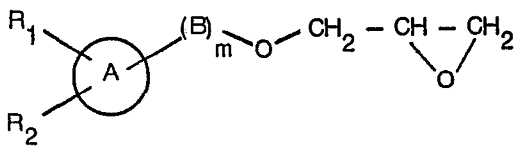

(II)

que l'on fait réagir dans un solvant sur une amine primaire de formule III:

$$R—NH_2 \qquad (III)$$

dans laquelle R à la même signification que pour la formule I, pour obtenir les dérivés cycloalcoyle-propanolamines de formule (I).

2. Procédé selon la revendication 1, caractérisé en ce que le catalyseur du type ammonium quaternaire est l'hydrogéno-sulfate de tétrabutylammonium.

3. Procédé selon l'une des revendications 1 et 2, caractérisé en ce que la base utilisée est la soude.

**Patentansprüche**

1. Verfahren zur Herstellung von Cycloalkoylpropanolaminen entsprechend der allgemeinen Formel I:

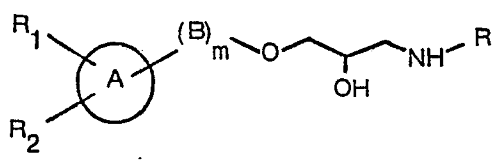

(I)

worin

A ein nichtaromatisches, carbozyklisches Radikal mit mindestens einem Ring darstellt,
$R_1$ ein Alkoyl- oder Acylradikal darstellt,
$R_2$ Wasserstoff oder ein Alkoylradikal darstellt,
B ein Methylenradikal darstellt,
m 0, 1 oder 2 ist, und
wenn m nicht 0 ist, $R_1$ Wasserstoff sein kann,
R ein Alkyl-, Alkenyl-, Alkinylradikal oder ein Aralkylradikal ist, und
wenn R ein ungesättigtes Radikal ist, $R_1$ Wasserstoff sein kann;

dadurch gekennzeichnet, daß Epichlorhydrin mit einem Alkohol der Formel IV:

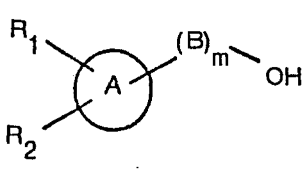

(IV)

zur Reaktion gebracht wird, worin $R_1$, $R_2$, A, B und m dieselbe Bedeutung wie in der Formel (I) haben, in Gegenwart einer Base in wässriger Lösung und eines Katalysators in Form einer quaternären Ammoniumverbindung zur Herstellung eines Epoxyds als Zwischenprodukt der Formel II:

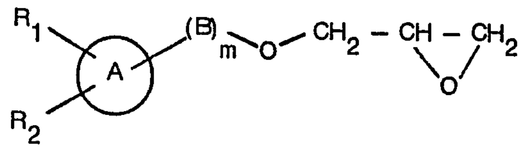

(II)

die man in einem Lösungsmittel mit einem primären Amin der Formel III:

$$R—NH_2 \qquad (III)$$

zur Reaktion bringt, worin R dieselbe Bedeutung hat wie in der Formel I, zu Herstellung von Cycloalkoylpropanolaminderivaten der Formel (I).

2. Verfahren nach Anspruch 1, dadurch gekennzeichnet, daß der Katalysator in Form eines quaternären Ammoniums Tetrabutylammoniumhydrogensulfat ist.

3. Verfahren nach einem der Ansprüche 1 und 2, dadurch gekennzeichnet, daß die verwendete Base Soda ist.

**Claims**

1. A process for the preparation of cycloalkylpropanolamines corresponding to the general formula I:

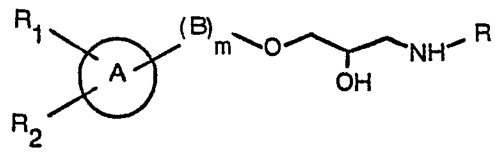

(I)

wherein

A represents a non aromatic, cyclic carbon radical, containing at least one ring.
$R_1$ represents an alkyl or acyl radical,
$R_2$ represents hydrogen or an alkyl radical,
B represents a methylene radical,
m represents 0, 1 or 2, and
when *m* does not represent 0, $R_1$ may represent hydrogen,
R represents an alkyl, alkenyl, alkynyl, or an aralkyl radical, and when R represents an unsaturated radical, $R_1$ may represent hydrogen,

characterised in that epichlorohydrin is reacted with an alcohol corresponding to formula IV:

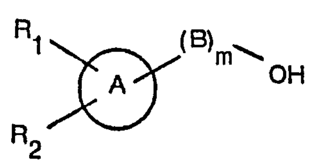

(IV)

wherein

$R_1$, $R_2$, A, B and m are as defined in formula (I), in the presence of a base in aqueous solution and in the presence of a catalyst of the quaternary ammonium type, to obtain the intermediate epoxide corresponding to formula II:

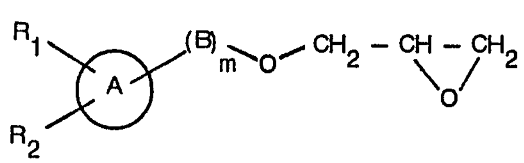

(II)

which is reacted in a solvent with a primary amine corresponding to formula III:

$$R—NH_2 \qquad (III)$$

wherein R is as defined in formula I, to obtain the cycloakyl propanolamine derivatives corresponding to formula (I).

2. A process according to claim 1, characterised in that the catalyst of the quaternary ammonium type is tetrabutyl ammonium hydrogen-sulphate.

3. A process according to one of claims 1 and 2, characterised in that the base which is used is soda.